(19) Europäisches Patentamt
European Patent Office
Office européen des brevets

(11) Numéro de publication : **0 511 072 A1**

(12) **DEMANDE DE BREVET EUROPEEN**

(21) Numéro de dépôt : **92401108.3**

(51) Int. Cl.$^5$ : **C07D 239/42,** A61K 31/505

(22) Date de dépôt : **21.04.92**

(30) Priorité : **24.04.91 FR 9105043**

(43) Date de publication de la demande : **28.10.92 Bulletin 92/44**

(84) Etats contractants désignés : **AT BE CH DE DK ES FR GB GR IT LI LU MC NL PT SE**

(71) Demandeur : **SYNTHELABO**
**22, Avenue Galilée**
**F-92350 Le Plessis Robinson (FR)**

(72) Inventeur : **George, Pascal**
**19, rue des Quatre Vents**
**F-78730 St. Arnoult en Yvelines (FR)**
Inventeur : **Manoury, Philippe**
**L'Orée de Verrières, 38, Avenue des Vaupépins**
**F-91370 Verrières le Buisson (FR)**
Inventeur : **Mangane, Michel**
**44, Avenue Marcel Cachin**
**F-92320 Chatillon s/Bagneux (FR)**
Inventeur : **Merly, Jean-Pierre**
**11, Avenue Jules Guesde**
**F-92330 Sceaux (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

(54) **Dérivés de 2-aminopyrimidine-4-carboxamide, leur préparation et leur application en thérapeutique.**

(57) Dérivés de 2-aminopyrimidine-4-carboxamide répondant à la formule générale (I)

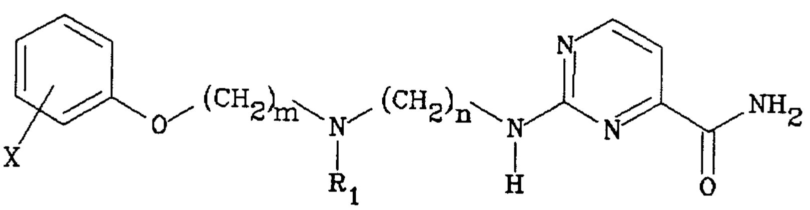

(I)

dans laquelle
m représente le nombre 2 ou 3,
n représente le nombre 2 ou 3,
$R_1$ représente un atome d'hydrogène ou un groupe méthyle, et
X représente un ou plusieurs atomes ou groupes choisis parmi les atomes d'hydrogène, de fluor et de chlore et les groupes méthoxy, éthoxy, méthyle et 1-méthyléthyle.
Application en thérapeutique.

EP 0 511 072 A1

La présente invention a pour objet des dérivés de 2-aminopyrimidine-4-carboxamide, leur préparation et leur application en thérapeutique.

Les composés de l'invention répondent à la formule générale (I)

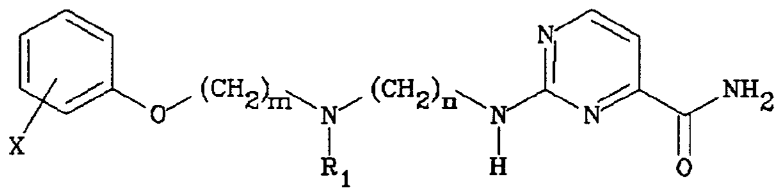

(I)

dans laquelle
m représente le nombre 2 ou 3,
n représente le nombre 2 ou 3,
$R_1$ représente un atome d'hydrogène ou un groupe méthyle, et
X représente un ou plusieurs atomes ou groupes choisis parmi les atomes d'hydrogène, de fluor et de chlore et les groupes méthoxy, éthoxy, méthyle et 1-méthyléthyle.

Les composés de l'invention peuvent se présenter à l'état de bases libres ou de sels d'addition à des acides pharmaceutiquement acceptables.

Conformément à l'invention, on peut préparer les composés de formule générale (I) selon le procédé illustré par le schéma 1 donné ci-après.

On fait réagir une amine de formule générale (II) (dans laquelle X, m, et $R_1$ sont tels que définis ci-dessus), éventuellement sous forme de chlorhydrate, avec un réactif halogéné de formule générale (III) (dans laquelle Y représente un atome d'halogène, R représente un groupe protecteur de l'amine, par exemple un groupe triphénylméthyle, et n est tel que défini ci-dessus). La réaction s'effectue dans un solvant aprotique tel que le N,N-diméthylformamide en présence d'une base minérale telle que le carbonate de potassium à une température de 40 à 80°C.

On obtient une diamine de formule générale (IV). On déprotège

Schéma 1

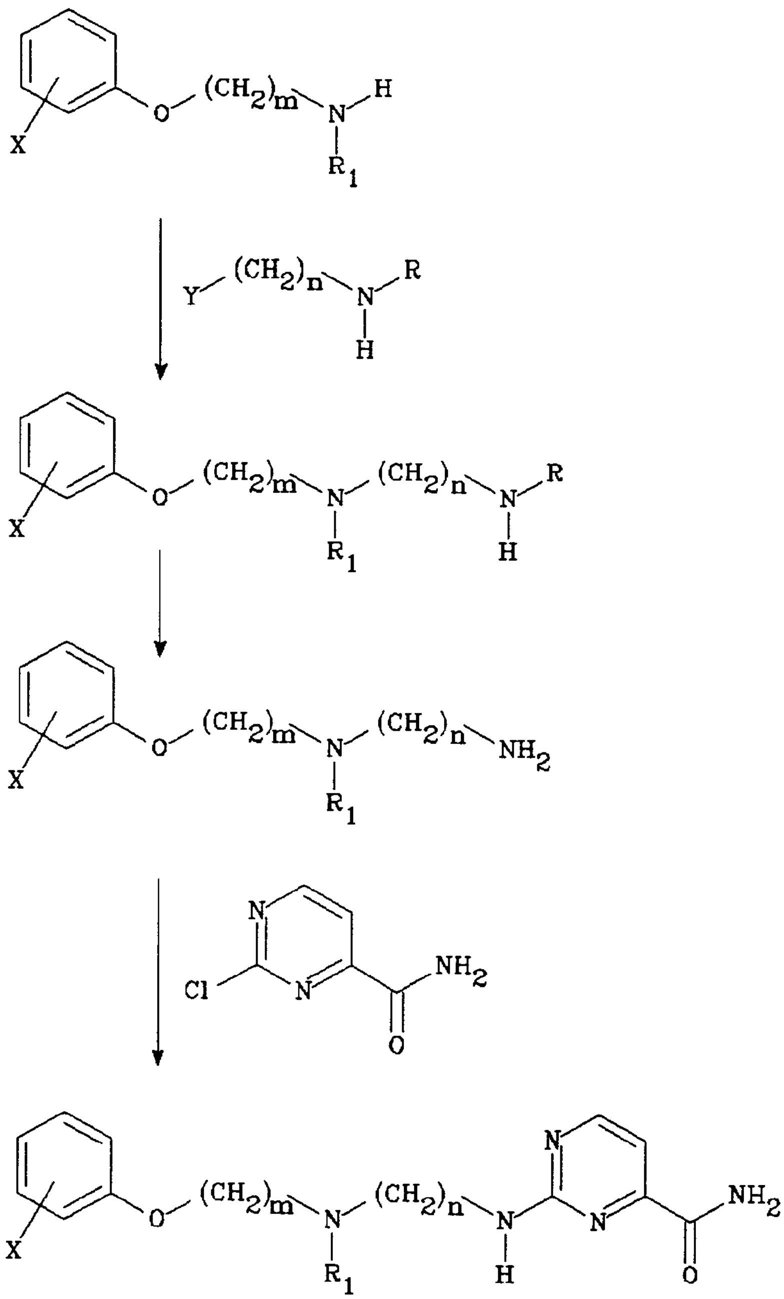

(II)

(III)

(IV)

(V)

(VI)

(I)

l'alkylamine terminale par traitement à l'acide chlorhydrique gazeux dans un alcool aliphatique, par exemple le méthanol, à une température de 0 à 60°C.

On obtient ainsi une diamine de formule générale (V) que l'on fait réagir avec le 2-chloropyrimidine-4-carboxamide de formule (VI) dans un solvant aprotique, par exemple le *N,N*-diméthylformamide , en présence d'une base, par exemple le carbonate de potassium à une température de 20 à 40°C, pour obtenir le dérivé de 2-aminopyrimidine-4-carboxamide de formule générale (I).

Les phénoxyalkylamines de formule générale (II) peuvent être obtenues par des méthodes analogues à celles décrites dans *Bull. Soc. Chim.* (1959) 839-849.

La 3-bromo-N-(triphénylméthyl)propanamine de formule générale (III) et le 2-chloropyrimidine-4-carboxamide sont décrits dans la demande de brevet EP-0 435 749.

Le réactif halogéné de formule générale (III) peut être préparé suivant le schéma 2, donné ci-dessous.

Schéma 2

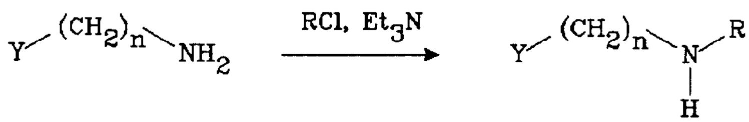


(VII) (III)

On fait réagir une l-halogénoalkylamine de formule générale (VII) avec un composé de formule RCl, en l'occurrence le chlorure de trityle (chlorure de triphénylméthyle), dans un solvant inerte halogéné tel que le dichlorométhane, en présence d'une base organique telle que la triéthylamine, à une température de 20 à 80°C.

Le 2-chloropyrimidine-4-carboxamide de formule (VI), peut être préparé selon le schéma 3, donné ci-dessous.

On traite le 2-chloropyrimidine-4-carbonitrile de formule (VIII) par l'acide chlorhydrique gazeux dans l'acide formique. Le 2-chloropyrimidine-4-carbonitrile est préparé selon la méthode décrite dans *J. Het. Chem.* (1964), 1, 130-133.

Schéma 3

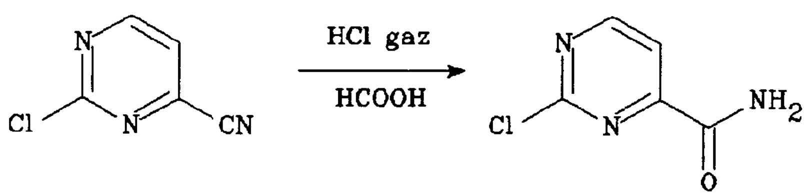


(VIII) (VI)

Les exemples suivants illustrent la préparation de quelques composés selon l'invention.

Les microanalyses élémentaires et les spectres IR et RMN confirment les structures des produits obtenus.

Exemple 1.

(*E*)-But-2-ènedioate de 2-[[3-[(2-phénoxyéthyl)amino]propyl]amino]pyrimidine-4-carboxamide.

1.1. N-(2-phénoxyéthyl)-N'-(triphénylméthyl)propane-1,3-diamine.

Dans un ballon tricol de 500 ml, on introduit sous argon 12,90 g (0,0743 mole) de chlorhydrate de 2-phénoxyéthylamine, 31,1 g (0,0817 mole) de N-trityl-3-bromo-propylamine, 25,7 g (0,186 mole) de carbonate de potassium et 150 ml de N,N-diméthylformamide.

On agite le mélange réactionnel à 90°C, pendant 16 heures puis le verse sur de l'eau glacée et on extrait à l'acétate d'éthyle. On lave la phase organique à l'eau, la sèche et la concentre sous pression réduite. On obtient une huile jaune que l'on purifie par chromatographie sur une colonne de silice avec un mélange dichlorométhane/méthanol 99/1 (rendement 42%).

1.2. Dichlorhydrate de N-(2-phénoxyéthyl)propane-1,3-diamine. Dans un ballon de 1 l, on introduit 12,7 g (0,029 mole) du composé 1.1. et 350 ml de méthanol. On fait barboter de l'acide chlorhydrique gazeux pendant 10 mn, en refroidissant par un mélange d'eau et de glace. On obtient une solution limpide, jaune clair. On laisse revenir le mélange à la température ambiante, puis on le porte à la température du reflux pendant 6 heures. On concentre partiellement le mélange sous pression réduite jusqu'à 100 ml. On laisse refroidir, et on obtient un solide blanc que l'on isole par filtration puis séchage.

F= 274-277°C (rendement 96,8%).

1.3. (*E*)-But-2-ènedioate de 2-[[3-[(2-phénoxyéthyl)amino]propyl]amino]pyrimidine-4-carboxamide.

Dans un ballon de 500 ml, on introduit sous argon, 4,0 g (0,0150 mole) du composé 1.2., 2,4 g (0,0153 mole) de 2-chloropyrimidine-4-carboxamide, 75 ml de N,N-diméthylformamide et un peu d'iodure de sodium. Enfin, on ajoute 7,25 g (0,0525 mole) de carbonate de potassium et on agite le mélange à 40°C, pendant 10 heures.

On traite le mélange réactionnel par un mélange d'eau et de glace et extrait à l'acétate d'éthyle. On lave la phase organique à l'eau, la sèche et la concentre sous pression réduite.

On obtient une huile que l'on purifie par chromatographie sur silice avec un mélange dichlorométhane/méthanol 94/6. On obtient un solide jaune que l'on dissout dans de l'acétate d'éthyle. On filtre et concentre le filtrat sous pression réduite.

On prépare le fumarate à partir de 1,73 g (0,00549 mole) de base dans 100 ml d'éthanol et 0,64 g (0,00549 mole) d'acide fumarique dans 70 ml d'éthanol. On obtient une solution limpide, jaune clair, que l'on concentre à froid à 90%. On obtient un solide blanc, que l'on fait recristalliser dans un mélange éthanol/méthanol 4/1 (rendement 31,8%).

F = 175-177,5°C.

Exemple 2.

(*E*)-But-2-ènedioate de 2-[[3-[(2-(2-méthoxyphénoxy)éthyl]méthylamino]propyl]amino]pyrimidine-4-carboxamide.

2.1. *N*-(2-(2-méthoxyphénoxy)éthyl]-*N*-méthyl-*N'*-(triphénylméthyl)propane-1,3-diamine.

Dans un ballon tricol de 500 ml, on introduit sous argon, 8,05 g (0,0370 mole) de chlorhydrate de *N*-méthyl-2-(2-méthoxyphénoxy)éthylamine, 15,5 g (0,0407 mole) de N-trityl-3-bromopropylamine, (0,0925 mole) de carbonate de potassium et 75 ml de *N,N*-diméthylformamide. On agite le mélange pendant 15,5 heures à 90°C. On traite le mélange réactionnel par un mélange d'eau et de glace et extrait à l'acétate d'éthyle. On lave la phase organique à l'eau, la sèche et la concentre sous pression réduite. On obtient 18,2 g d'une huile orange que l'on chromatographie sur silice avec un mélange de dichlorométhane/méthanol 98/2. On obtient 13,7 g d'huile (rendement 77%).

2.2. *N*-[2-(2-méthoxyphénoxy)éthyl]-*N*-méthylpropane-1,3-diamine.

Dans un ballon de 1 l, on introduit 12,9 g (0,0268 mole) du composé 2.1. et 250 ml de méthanol. On fait barboter de l'acide chlorhydrique gazeux pendant 15 mn, en refroidissant par un mélange d'eau et de glace. On laisse revenir le mélange à la température ambiante, puis le porte à la température du reflux pendant 7,5 heures. On concentre le mélange à sec, reprend le résidu dans de l'éthanol et concentre à nouveau. On revient à la base par une extraction acide-base : on reprend l'huile par un mélange d'eau et d'acide chlorhydrique dilué et on extrait à l'éther. On traite la phase aqueuse par de la soude et extrait au dichlorométhane. On lave la phase organique à l'eau, la sèche et la concentre sous pression réduite. On obtient 5,6 g d'une huile jaune (rendement 87,5%).

2.3. (*E*)-But-2-ènedioate de 2-[[3-[(2-(2-méthoxyphénoxy)éthyl]méthylamino]propyl]amino]pyrimidine-4-carboxamide.

Dans un ballon de 500 ml, on introduit sous argon, 5,55 g (0,0233 mole) du composé 2.2, 3,75 g (0,0238 mole) de 2-chloropyrimidine-4-carboxamide, 120 ml de *N,N*-diméthylformamide et quelques cristaux d'iodure de sodium. Enfin, on ajoute 4,83 g (0,0350 mole) de carbonate de potassium et agite le mélange à 50°C, pendant 8,5 heures.

On traite le mélange réactionnel par un mélange d'eau et de glace et extrait à l'acétate d'éthyle. On lave la phase organique à l'eau, sèche et concentre sous pression réduite. On obtient 6,0 g d'une huile jaune que l'on chromatographie sur silice par un mélange de dichlorométhane/méthanol 92/8. On prépare le fumarate à partir de 6,0 g (0,0167 mole) de base dans 100 ml d'éthanol et de 1,94 g (0,0167 mole) d'acide fumarique dans 200 ml d'éthanol.

On concentre la solution limpide, jaune clair et obtient une huile jaune. On ajoute de l'acétate d'éthyle et triture à chaud. On obtient un solide blanc que l'on fait recristalliser dans de l'éthanol (rendement 49,6%).

F = 133 - 136°C.

Exemple 3.

(*E*)-But-2-ènedioate de 2-[[3-[[3-[5-méthyl-2-(1-méthyléthyl)-phénoxy]propyl]méthylamino]propyl]amino]pyrimidine-4-carboxamide.

3.1. *N*-méthyl-*N*-[3-[5-méthyl-2-(1-méthyléthyl)phénoxy]propyl]-*N'*-(triphénylméthyl)propane-1,3-diamine.

Dans un ballon tricol de 500 ml, sous argon, on introduit 8,6 g (0,0334 mole) de chlorhydrate de N-méthyl-3-[5-méthyl-2-(1-méthyléthyl]phénoxy]propylamine, 14,0 g (0,0367 mole) de *N*-trityl-3-bromopropylamine, 11,55 g (0,0835 mole) de carbonate de potassium et 70 ml de *N,N*-diméthylformamide. On agite le mélange réactionnel à 90-100°C, pendant 15,5 heures puis le traite par un mélange d'eau et de glace, et on extrait à l'acétate d'éthyle. On lave la phase organique à l'eau, la sèche et la concentre sous pression réduite. On obtient une huile jaune que l'on chromatographie sur silice avec un mélange de dichlorométhane/méthanol 9/1 (rendement 54,6%).

3.2. Dichlorhydrate de N-méthyl-N-[3-[5-méthyl-2-(1-méthyléthyl)phénoxy]propyl]propane-1,3-diamine.

Dans un ballon de 500 ml, on introduit 9,3 g (0,0179 mole) du composé 3.1. et 180 ml de méthanol. On fait barboter de l'acide chlorhydrique gazeux pendant 20 mn, en refroidissant par un mélange d'eau et de glace. On laisse revenir le mélange réactionnel à la température ambiante, puis le porte à la température du reflux pendant 7 heures. On concentre le mélange à sec, obtient un solide crème, huileux, que l'on reprend dans de l'éthanol. On concentre, à nouveau, à 90%. On obtient une huile jaune, à laquelle on ajoute de l'acétate d'éthyle et triture. On obtient un solide blanc amorphe (rendement 90,5%).

3.3. (*E*)-But-2-ènedioate de 2-[[3-[[3-[5-méthyl-2-(1-méthyléthyl)phénoxy]propyl]méthylamino]propyl]amino]pyrimidine-4-carboxamide.

Dans un ballon tricol de 500 ml, on introduit sous argon, 5,6 g (0,0159 mole) du composé 3.2., 2,6 g (0,0164 mole) de 2-chloropyrimidine-4-carboxamide, 80 ml de *N,N*-diméthylformamide et quelques cristaux d'iodure de sodium. Enfin, on ajoute 7,7 g (0,0557 mole) de carbonate de potassium et agite le mélange réactionnel à 50°C, pendant 8,5 heures puis le traite par un mélange d'eau et de glace. On extrait à l'acétate d'éthyle, lave la phase organique à l'eau, sèche et concentre sous pression réduite. On obtient une huile jaune que l'on utilise telle quelle.

On prépare le fumarate à partir de 6,3 g (0,0158 mole) de base dans 100 ml d'éthanol et de 1,83 g (0,0158 mole) d'acide fumarique dans 180 ml d'éthanol. On concentre presque totalement la solution limpide obtenue, ajoute de l'acétate d'éthyle et triture. On obtient un solide blanc que l'on fait recristalliser dans de l'éthanol (rendement 68,3%). F = 152,5 - 154,5°C.

Le tableau qui suit illustre les propriétés physiques de quelques composés selon l'invention. Dans la colonne "Sel", "fum." désigne un fumarate acide, "½fum." désigne un fumarate neutre, et 2HCl désigne un dichlorhydrate.

Tableau

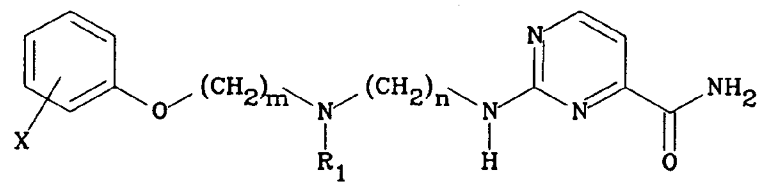

(I)

| N° | X | m | n | $R_1$ | Sel | F(°C) |
|---|---|---|---|---|---|---|
| 1 | H | 2 | 3 | H | fum. | 175-177,5 |
| 2 | H | 2 | 3 | $CH_3$ | fum. | 149-151,5 |
| 3 | 2-$OCH_3$ | 2 | 3 | H | fum. | 158,5-161 |
| 4 | 2-$OCH_3$ | 2 | 3 | $CH_3$ | fum. | 133-136 |
| 5 | 2-$OCH_3$, 5-Cl | 2 | 3 | H | ½fum. | 179,5-182 |
| 6 | 2-$OCH_3$, 5-Cl | 2 | 3 | $CH_3$ | fum. | 157,5-160 |
| 7 | 2-i$C_3H_7$ | 2 | 3 | H | fum. | 175-178 |
| 8 | 2-i$C_3H_7$ | 2 | 3 | $CH_3$ | fum. | 146-149 |
| 9 | 2-i$C_3H_7$, 5-$CH_3$ | 2 | 3 | H | fum. | 186-188,5 |
| 10 | 2-i$C_3H_7$, 5-$CH_3$ | 2 | 3 | $CH_3$ | 2HCl | 141-142 |
| 11 | 2-i$C_3H_7$, 5-$CH_3$ | 3 | 3 | $CH_3$ | fum. | 152,5-154,5 |
| 12 | 4-F | 2 | 3 | H | fum. | 164,5-167 |
| 13 | 4-F | 2 | 3 | $CH_3$ | fum. | 140-142 |
| 14 | 2-$OCH_3$, 5-F | 2 | 3 | $CH_3$ | fum. | 129,5-132,5 |
| 15 | 4-F | 3 | 2 | $CH_3$ | fum. | 166-169 |
| 16 | 2-i$C_3H_7$, 5-$CH_3$ | 3 | 2 | $CH_3$ | fum. | 199-202 |
| 17 | 2-$OCH_3$, 5-F | 2 | 3 | H | fum. | 171-173 |
| 18 | 2-$OC_2H_5$ | 2 | 3 | $CH_3$ | fum. | 123-125,5 |

Les composés de l'invention ont fait l'objet d'études quant à leur activité antagoniste des récepteurs $\alpha_1$-adrénergiques au niveau du bas appareil urinaire.

Leur activité *in vitro* a été étudiée sur l'urètre isolé de lapin.

On prépare des anneaux d'urètre de lapin adulte selon la méthode de Ueda et al., *Eur. J. Pharmacol.*, (1984), 103, 249-254, puis, après sensibilisation à la noradrénaline, on détermine la courbe concentration-réponse à la phényléphrine, en absence et en présence de composé à étudier.

On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.

Les $pA_2$ des composés sont de l'ordre de 5,5 à 9.

L'activité *in vivo* des composés de l'invention a été étudiée quant à leur effet sur l'hypertonie urétrale engendrée par la stimulation des fibres sympathiques du nerf hypogastrique chez le chat anesthésié.

On anesthésie des chats mâles adultes au pentobarbital sodique, et on les prépare selon la méthode de Theobald, *J. Auton. Pharmac.*, (1983), 3, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.

On évalue la puissance de l'antagonisme $\alpha_1$-adrénergique de chaque composé par calcul de la $DI_{50}$, dose qui inhibe de 50% l'hypertonie urétrale. Les $DI_{50}$ des composés de l'invention sont de l'ordre de 0,01 à 3 mg/kg.

Les résultats des essais montrent que les composés de l'invention montrent, *in vitro*, une activité antagoniste des récepteurs $\alpha_1$-adrénergiques des muscles lisses du bas appareil urinaire (urètre) stimulés par un agoniste $\alpha_1$-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système a-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 100 mg de substance active.

## Revendications

**1.** Dérivés de 2-aminopyrimidine-4-carboxamide répondant à la formule générale (I)

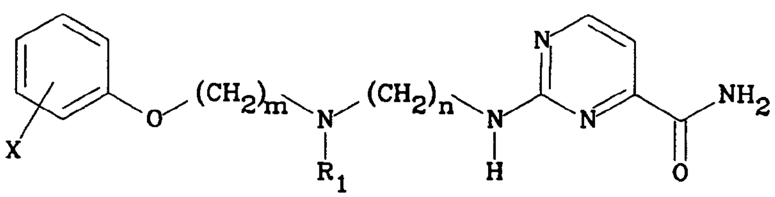

(I)

dans laquelle
m représente le nombre 2 ou 3,
n représente le nombre 2 ou 3,
$R_1$ représente un atome d'hydrogène ou un groupe méthyle, et
X représente un ou plusieurs atomes ou groupes choisis parmi les atomes d'hydrogène, de fluor et de chlore et les groupes méthoxy, éthoxy, méthyle et 1-méthyléthyle,
ainsi que leurs sels d'addition aux acides pharmaceutiquement acceptables.

**2.** Composé selon la revendication 1, en l'espèce le 2-[[3-[[2-(2-méthoxyphénoxy)éthyl]méthylamino]propyl]amino]pyrimidine-4-carboxamide.

**3.** Procédé de préparation de dérivés selon la revendication 1, procédé caractérisé en ce que l'on fait réagir une amine de formule générale (II)

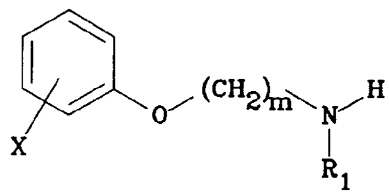

(II)

(dans laquelle X, m, et $R_1$ sont tels que définis ci-dessus), éventuellement sous forme de chlorhydrate, avec un réactif halogéné de formule générale (III)

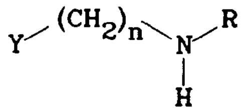

(III)

(dans laquelle Y représente un atome d'halogène, R représente un groupe protecteur de l'amine et n est tel que défini ci-dessus) dans un solvant aprotique, en présence d'une base minérale, à une température de 40 à 80°C, pour obtenir une diamine de formule générale (IV)

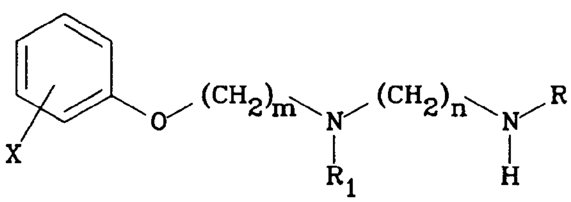

(IV)

puis on déprotège l'alkylamine terminale par traitement à l'acide chlorhydrique gazeux dans un alcool aliphatique, à une température de 0 à 60°C, pour obtenir une diamine de formule générale (V)

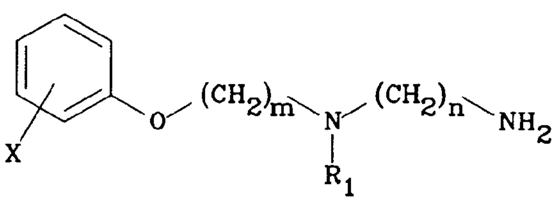

(V)

que l'on fait réagir avec le 2-chloropyrimidine-4-carboxamide dans un solvant aprotique, en présence d'une base, à une température de 20 à 40°C.

4. Médicament caractérisé en ce qu'il consiste en un composé selon l'une des revendications 1 et 2.

5. Composition pharmaceutique caractérisée en ce qu'elle contient un composé selon l'une des revendications 1 et 2, en association avec tout excipient approprié.

**Revendications pour les Etats contractants suivants : ES, GR**

1. Procédé de préparation de composés répondant à la formule générale (I)

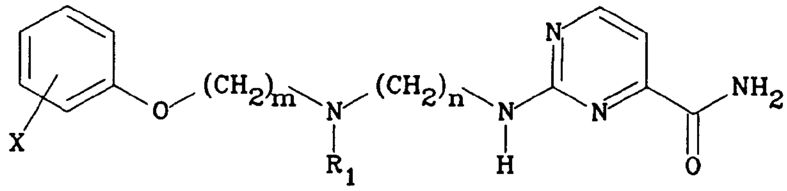

(I)

dans laquelle
m représente le nombre 2 ou 3,
n représente le nombre 2 ou 3,
$R_1$ représente un atome d'hydrogène ou un groupe méthyle, et
X représente un ou plusieurs atomes ou groupes choisis parmi les atomes d'hydrogène, de fluor et de chlore et les groupes méthoxy, éthoxy, méthyle et 1-méthyléthyle,
procédé caractérisé en ce que l'on fait réagir une amine de formule générale (II)

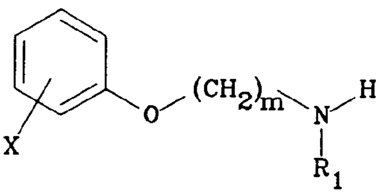

(II)

(dans laquelle X, m, et $R_1$ sont tels que définis ci-dessus), éventuellement sous forme de chlorhydrate, avec un réactif halogéné de formule générale (III)

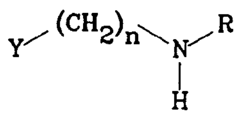

(III)

(dans laquelle Y représente un atome d'halogène, R représente un groupe protecteur de l'amine et n est tel que défini ci-dessus ) dans un solvant aprotique, en présence d'une base minérale, à une température de 40 à 80°C, pour obtenir une diamine de formule générale (IV)

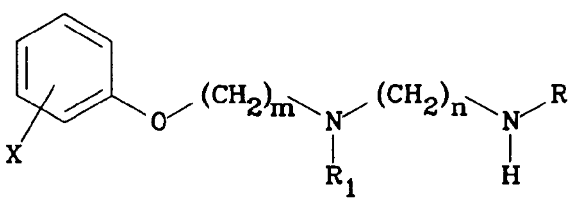

(IV)

puis on déprotège l'alkylamine terminale par traitement à l'acide chlorhydrique gazeux dans un alcool aliphatique, à une température de 0 à 60°C, pour obtenir une diamine de formule générale (V)

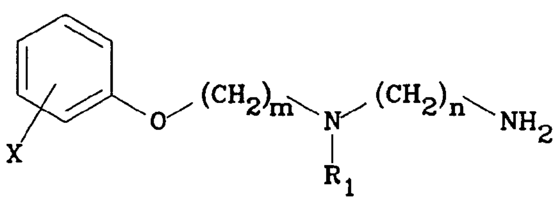

(V)

que l'on fait réagir avec le 2-chloropyrimidine-4-carboxamide dans un solvant aprotique, en présence d'une base, à une température de 20 à 40°C.

Office européen des brevets

# RAPPORT DE RECHERCHE EUROPEENNE

Numero de la demande

EP 92 40 1108

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-0 230 402 (DOMPE FARMACEUTICI)<br>* page 1 - page 4; revendications 1-11 *<br>----- | 1-5 | C07D239/42<br>A61K31/505 |

| DOMAINES TECHNIQUES RECHERCHES (Int. Cl.5 ) |
|---|
| C07D |

Le présent rapport a été établi pour toutes les revendications

| Lieu de la recherche | Date d'achèvement de la recherche | Examinateur |
|---|---|---|
| LA HAYE | 11 JUIN 1992 | FRANCOIS J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)